(19) Europäisches Patentamt
European Patent Office
Office européen des brevets

(11) Veröffentlichungsnummer : **0 079 004**
**B1**

(12) # EUROPÄISCHE PATENTSCHRIFT

(45) Veröffentlichungstag der Patentschrift : **16.04.86**

(51) Int. Cl.⁴ : **C 07 C120/06**// C07C121/34, C07C121/76

(21) Anmeldenummer : **82109997.5**

(22) Anmeldetag : **29.10.82**

(54) **Verfahren zur Herstellung von Acylcyaniden.**

(30) Priorität : **11.11.81 DE 3144792**
**15.07.82 DE 3226425**

(43) Veröffentlichungstag der Anmeldung : **18.05.83 Patentblatt 83/20**

(45) Bekanntmachung des Hinweises auf die Patenterteilung : **16.04.86 Patentblatt 86/16**

(84) Benannte Vertragsstaaten : **AT BE CH DE FR GB IT LI NL**

(56) Entgegenhaltungen :
**DE-A- 2 614 241**
**SYNTHESIS, Nr. 3, März 1979, Seiten 204-205, Georg Thieme Publishers K. HERRMANN et al.: "Synthese von Acylcyaniden(alpha-Oxonitrilen) mit Cyanotrimethylsilan"**

(73) Patentinhaber : **BAYER AG**
**Konzernverwaltung RP Patentabteilung**
**D-5090 Leverkusen 1 Bayerwerk (DE)**

(72) Erfinder : **Findeisen, Kurt, Dr.**
**In der Follmühle 10**
**D-5068 Odenthal 2 (DE)**
Erfinder : **Kranz, Eckart, Dr.**
**Am Acker 9**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 079 004 B1

Jouve, 18, rue St-Denis, 75001 Paris, France

**Beschreibung**

Die vorliegende Erfindung betrifft ein neues Verfahren zur Herstellung von teilweise bekannten Acylcyaniden, welche als Ausgangsstoffe zur Synthese von Herbiziden verwendet werden können.

Es ist bereits bekannt geworden, Benzoesäureanhydrid mit Kaliumcyanid in äquivalenter Menge umzusetzen, wobei in geringer Ausbeute (ca. 10 % d. Th.) Benzoylcyanid erhalten wird (vgl. Liebigs Annalen der Chemie 287, S. 306 (1895)). Das Benzoylcyanid muß der als Hauptprodukt gebildeten zähen, sehr stark verharzten, dunkelbraunen Masse mit Ether entzogen werden. Dies Verfahren ist technisch völlig ungeeignet, denn nicht nur die Extraktion mit Ether führt zu technisch unüberwindlichen Schwierigkeiten, sondern für die sehr stark verharzten, dunkelbraunen Massen besteht keine weitere Verwendung. Es handelt sich hierbei also lediglich um eine mögliche Bildungsweise für Benzoylcyanid.

Weiterhin ist bekannt geworden, daß man Acylcyanide, ausgehend von Carbonsäureanhydriden, in guten Ausbeuten erhält, wenn man die Carbonsäureanhydride entweder mit wasserfreier Blausäure oderunter bestimmten, gegenüber der obengenannten Literaturstelle verbesserten Verfahrensbedingungen — mit Alkalicyaniden umsetzt (vgl. z. B. DE-OS 26 14 241 und DE-OS 26 42 199).

Die zuletzt genannten Verfahren sind aber nicht frei von Nachteilen : Bei Verwendung von Alkalicyaniden entsteht während der Reaktion ein Salzrückstand, der nur schwer zu entfernen ist, außerdem den Wärmeübergang erschwert. Bei Verwendung von Blausäure müssen insbesondere wegen der extremen Toxizität und wegen des niedrigen Siedepunktes die bekannten, aufwendigen Vorsichtsmaßnahmen getroffen werden.

Ferner ist aus Synthesis, Nr. 3 (1979), Seiten 204-205, die Herstellung von aliphatischen Acylcyaniden durch Umsetzung von Acylhalogeniden mit Trimethylsilylcyanid bekannt ; dieses Verfahren liefert jedoch im allgemeinen nur relativ mäßige Ausbeuten.

Es wurde nun gefunden, daß man Acylcyanide der Formel

$$R-\overset{\overset{\displaystyle O}{\|}}{C}-CN \qquad (I)$$

in welcher R für gegebenenfalls substituiertes Alkyl mit 3 bis 8 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6-gliedrigen heterocyclischen Rest steht, der zusätzlich noch mit einem Benzolring anelliert sein kann,

in sehr hoher Ausbeute und ausgezeichneter Reinheit erhält, wenn man Carbonsäureanhydride der Formel

$$R-\overset{\overset{\displaystyle O}{\|}}{C}-O-\overset{\overset{\displaystyle O}{\|}}{C}-R \qquad (II)$$

in welcher R die oben angegebene Bedeutung hat, mit Trimethylsilylcyanid, $(CH_3)_3Si—CN$ (III), gegebenenfalls in Gegenwart eines Katalysators und gegebenenfalls in Gegenwart eines Verdünnungsmittels, bei Temperaturen zwischen 50 und 250 °C umsetzt.

Es ist als ausgeprochen überraschend zu bezeichnen, daß Acylcyanide der Formel (I) nach dem neuartigen erfindungsgemäßen Verfahren in hoher Ausbeute zugänglich sind und durch einfache Destillation gereinigt werden können. Das neue Verfahren vermeidet gleichzeitig die genannten Nachteile der vorbekannten Verfahren. Das neue Verfahren ist nicht auf die Synthese weniger bestimmter Verbindungen beschränkt, sondern es läßt sich sehr breit anwenden. — Wie insbesondere ein Vergleich mit der oben zitierten Literaturstelle Synthesis, Nr. 3 (1979), S. 204-205, zeigt, werden mit dem vorbekannten Verfahren nur relativ mäßige Ausbeuten an aliphatischen Acylcyaniden erzielt (vorwiegend unter 70 % d. Th.), während das erfindungsgemäße Verfahren generell hohe Ausbeuten an Acylcyaniden liefert (vorwiegend über 90 % d. Th.), wobei dies sowohl für aliphatische als auch cycloaliphatische und insbesondere aromatische Acylcyanide gilt.

Die im Verlauf der Reaktion entstehenden Trimethylsilylester können entweder durch Destillation oder auf chemischen Wege (s. unten) von den gebildeten Acylcyaniden getrennt werden.

Setzt man Benzoesäureanhydrid mit Trimethylsilylcyanid um, so kann der Reaktionsverlauf durch das folgende Formelschema wiedergegeben werden :

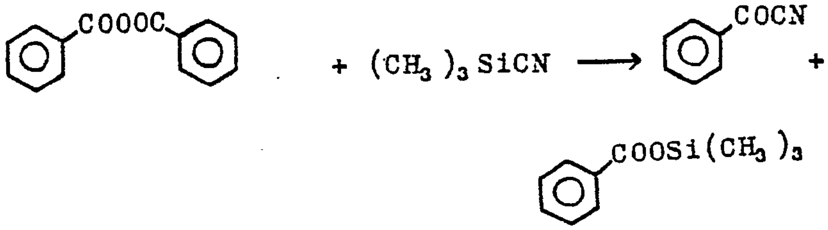

Die als Ausgangsstoffe verwendeten Carbonsäureanhydride sind durch die Formel (II) allgemein definiert. In dieser Formel steht R vorzugsweise für geradkettiges oder verzweigtes Alkyl mit 3 bis 4 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Cyano und/oder Halogen (wie Fluor, Chlor, Brom oder Jod). Ferner steht R vorzugsweise für gegebenenfalls durch Alkyl, Alkoxy oder Carbalkoxy mit jeweils bis zu 4 Kohlenstoffatomen, Nitro, Cyano und/oder Halogen (wie Fluor, Chlor und Brom) substituiertes Cycloalkyl mit 5 oder 6 Kohlenstoffatomen im Ringsystem. Weiterhin steht R vorzugsweise für gegebenenfalls durch Alkyl, Alkoxy oder Carbalkoxy mit jeweils bis zu 4 Kohlenstoffatomen, Nitro und/oder Halogen (wie Fluor, Chlor und Brom) substituiertes Aryl, insbesondere Phenyl oder Naphthyl. Schließlich steht R vorzugsweise für gegebenenfalls durch Alkyl, Alkoxy oder Carbalkoxy mit jeweils bis zu 4 Kohlenstoffatomen, Nitro, Cyano und/oder Halogen (wie Fluor, Chlor oder Brom) substituierte 5- oder 6-gliedrige heterocyclische Reste, die 1 bis 3 Heteroatome wie Sauerstoff, Schwefel und/oder Stickstoff im Ring enthalten können und außerdem mit einem Benzolring anelliert sein können.

Als Beispiele für insbesondere in Frage kommende heterocyclische Reste seien genannt : Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Als bevorzugte Beispiele für Carbonsäureanhydride der Formel (II) seien im einzelnen genannt : Pivalinsäureanhydrid, Cyclohexancarbonsäureanhydrid, Benzoesäureanhydrid, m-Chlorbenzoesäureanhydrid, 3,5-Dichlorbenzoesäureanhydrid, Naphthalin-1-carbonsäureanhydrid, 1-Phenyl-5-pyrazolon-3-Carbonsäureanhydrid. Als im Rahmen dieser Erfindung besonders bevorzugte Carbonsäureanhydride seien die aromatischen Carbonsäureanhydride, insbesondere das Benzoesäureanhydrid, sowie das Pivalinsäureanhydrid genannt.

Die Carbonsäureanhydride der Formel (II) sind bekannt oder sind nach allgemein bekannten Verfahren herstellbar.

Das Trimethylsilylcyanid, $(CH_3)_3SiCN$, ist gleichfalls bekannt (vgl. z. B. Synthesis 1979, S. 522 und 523, ferner DE-OS 30 18 821).

Als Verdünnungsmittel, die bei der Durchführung des erfindungsgemäßen Verfahrens eingesetzt werden können, kommen alle inerten organischen Lösungsmittel, die weder mit den Carbonsäureanhydriden noch mit dem Trimethylsilylcyanid eine chemische Reaktion eingehen, in Betracht. Solche Lösungsmittel sind beispielsweise die Xylole wie o-Xylol, Chlorbenzol, o-Dichlorbenzol, die Trichlorbenzole, Nitrobenzol, Tetramethylensulfon. Als Verdünnungsmittel besonders geeignet ist ein Überschuß an Carbonsäureanhydrid (II). — Prinzipiell ist es jedoch auch möglich, die erfindungsgemäße Umsetzung ohne Verdünnungsmittel durchzuführen.

Die Reaktionstemperatur kann in einem größeren Bereich variiert werden. Im allgemeinen arbeitet man bei Temperaturen zwischen 50 und 250 °C, vorzugsweise zwischen 70 und 230 °C, insbesondere zwischen 80 und 200 °C.

Die Reaktion wird im allgemeinen bei Normaldruck durchgeführt. Bei Verwendung von niedrigsiedenden, aliphatischen Carbonsäureanhydriden ist jedoch ein leichter Überdruck günstig, dann im allgemeinen bis zu 10 bar, vorzugsweise von 2 bis 6 bar.

Durch Zusatz katalytischer Mengen Lewis-Säure kann die Umsetzung beschleunigt werden. Als geeignete Lewis-Säuren seien beispielhaft genannt : Zinkchlorid, Zinkjodid, Zinkcyanid, Kupfer-I-cyanid, Aluminium-chlorid.

Mit besonderem Vorteil kann die Umsetzung auch durch Zusatz von Basen katalysiert werden. Geeignete Basen sind insbesondere tertiäre Amine wie Triethylamin oder 1,4-Diazabicyclo-(2.2.2)-octan, Salze von schwachen Carbonsäuren wie z. B. Natriumacetat, Natrium-cyanid, Kaliumcyanid oder Natriumhydrogencarbonat, ferner Alkoholate wie Natriummethylat oder Kalium-t-butylat.

Bei der Druchführung des erfindungsgemäßen Verfahrens setzt man im allgemeinen stöchiometrische Mengen an Carbonsäureanhydrid mit Trimethylsilylcyanid um. Das Säureanhydrid kann jedoch auch im Überschuß, vorteilhaft dann sogar als Lösungsmittel verwendet werden.

Der Katalysator wird im allgemeinen in Mengen von 0,001-0,1 Mol, vorzugsweise von 0,005-0,05 Mol, pro Mol Carbonsäureanhydrid (II) bzw. Trimethylsilylcyanid (III) eingesetzt.

Die Aufarbeitung erfolgt nach beendeter Umsetzung üblicherweise durch Destillation und gegebenenfalls Umkristallisation.

Auch in der Gasphase kann das Gemisch aus Carbonsäureanhydrid und Trimethylsilylcyanid erfindungsgemäß umgesetzt werden.

In einer besonderen Ausführungsform kann das erfindungsgemäße Verfahren auch kontinuierlich ausgestaltet werden.

Dei bei dem erfindungsgemäßen Verfahren neben den Acylcyaniden (I) gebildeten Carbonsäuretrimethylsilylester der Formel (IV) können nach einem nicht zum Stand der Technik gehörenden Verfahren (welches Gegenstand der europäischen Patentanmeldung 0 079 005 ist) durch Umsetzung mit den von der jeweils gleichen Carbonsäure (R-COOH) abgeleiteten Säurechloriden der Formel R-COCl(V) in die Ausgangs-Anhydride der Formel R-CO-O-CO-R (II) zurückverwandelt werden. Als weiteres Reaktionsprodukt wird hierbei Trimethylsilylchlorid, $(CH_3)_3SiCl$ (VI), gebildet, welches wiederum zur Herstellung von Trimethylsilylcyanid (III) eingesetzt werden kann :

$$R\text{-}COOSi(CH_3)_3 + RCOCl \longrightarrow R\text{-}CO\text{-}O\text{-}CO\text{-}R + (CH_3)_3SiCl$$

(IV) (V) (II) (VI)

$$(CH_3)_3SiCl \longrightarrow (CH_3)_3SiCN \quad ;$$

(VI) (III)

R hat hierin jeweils die oben angegebene Bedeutung.

Wenn die Siedepunkte der bei Durchführung des erfindungsgemäßen Verfahrens gebildeten Acylcyanide (I) und der Trimethylsilylester (IV) zu dicht beieinander liegen, kann es besonders zweckmäßig sein, die letzteren durch Umsetzung des Reaktionsgemisches mit dem entsprechenden Säurechlorid in die Ausgangs-Anhydride umzuwandeln, wodurch man ein destillativ sehr leicht auftrennbares Produktgemisch erhält, welches im wesentlichen aus Carbonsäureanhydrid (II), dem gewünschten Acylcyanid (I) und Trimethylsilylchlorid (VI) besteht.

Die nach dem erfindungsgemäßen Verfahren herstellbaren Acylcyanide der Formel (I) sind wertvolle Ausgangsstoffe z. B. zur Synthese von 1,2,4-Triazin-5-onen, welche hervorragende herbizide Eingeschaften besitzen (vgl. z. B. Deutsche Offenlegungsschrift 2 224 161).

So läßt sich beispielsweise das 3-Methyl-4-amino-6-phenyl-1,2,4-triazin-5-on (common name : Metamitron) der Formel

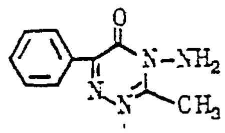

ausgehend von Benzoylcyanid nach einem bekannten Verfahren herstellen, indem man in einer ersten Stufe Benzoylcyanid in Gegenwart von konzentrierter Salzsäure mit Ethanol umsetzt und den dabei entstehenden Phenylglyoxylsäureethylester in einer zweiten Stufe mit Acetylhydrazin zur Reaktion bringt, wobei sich 1-Phenylglyoxylsäureethylester-2-acetylhydrazon bildet, das in einer dritten Stufe mit Hydrazinhydrat in Gegenwart von Pyridin in das oben erwähnte Endprodukt überführt wird.

Das erfindungsgemäß erhältliche Pivaloylcyanid kann nach ebenfalls bekannten Verfahren z. B. in den herbiziden Wirkstoff 3-Methylthio-4-amino-6-tert.-butyl-1,2,4-triazin-5(4H)-on (common name : Metribuzin) um gewandelt werden (vgl. z. B. DE-PS 17 95 784, DE-OS 27 33 180, US 4 175 188, DE-OS 30 02 203, DE-OS 30 03 541, DE-OS 30 09 043).

Die nachfolgenden Beispiele sollen zur weiteren Erläuterung der Erfindung dienen.

Beispiel 1

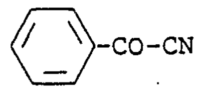

In einem mit Rührer, Thermometer, Rückflußkühler und Tropftrichter bestückten 500 ml-Vierhalskolben werden 226 g Benzoesäureanhydrid (1 Mol) auf 160 °C erhitzt und innerhalb von zwei Stunden werden 104 g Trimethylsilylcyanid (1,05 Mol) zugetropft. Nach beendeter Umsetzung wird die Innentemperatur kurz auf 200 °C gesteigert, anschließend wird über eine Kolonne fraktioniert destilliert. Ausbeute : 126 g (= 96 % d. Th.) Benzoylcyanid ; Schmelzpunkt : 31 °C.

Beispiel 2

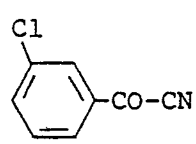

Wie zuvor beschrieben, werden 295 g 3-Chloro-benzoesäureanhydrid (1 Mol) auf 150 °C erhitzt und mit 1 g Zinkchlorid versetzt. Innerhalb von 90 Minuten tropft man 99 g Trimethylsilylcyanid (1 Mol) bei dieser Temperatur zu. Die Innentemperatur wird bis zur vollständigen Umsetzung kurz bei 180 °C gehalten, anschließend wird fraktioniert destilliert.

Ausbeute : 154 g 3-Chlorbenzoylcyanid (= 93 % d. Th.) ;

Siedepunkt 118-120 °C bei 18 mbar.

### Beispiel 3

$(CH_3)_3C\text{-}CO\text{-}CN$

Wie zuvor beschrieben, werden 186 g Pivalinsäureanhydrid (1 Mol) auf 180 °C erwärmt, mit 0,3 g Aluminiumchlorid versetzt und innerhalb von drei Stunden werden 99 g Trimethylsilylcyanid (1 Mol) zugetropft. Die Innentemperatur sinkt dabei allmählich auf 130 °C ab. Eine IR-spektroskopische Untersuchung zeigt, daß kein Pivalinsäureanhydrid mehr vorhanden ist. Die Ausbeute an Pivaloylcyanid nach Gaschromatogramm beträgt 98 % der Theorie.

Aufarbeitung :

Zur leichteren Trennung des Pivaloylcyanids vom Pivalinsäuretrimethylsilylester werden bei 110 °C innerhalb von drei Stunden 120,5 g Pivalinsäurechlorid (1 Mol) in die obige Reaktionsmischung getropft. Man rührt dreißig Minuten nach und destilliert fraktioniert.

Ausbeute : 105,5 g Pivaloylcyanid (≙ 96 % d. Th.) ;

Siedepunkt 121 °C bei 990 mbar.

Daneben wurden Trimethylsilylchlorid und Pivalinsäure-anhydrid in nahezu quantitativer Ausbeute erhalten.

Analog zu den Beispielen 1-3 können auch die in der folgenden Tabelle aufgeführten Acylcyanide hergestellt werden :

Tabelle 1

| Bsp. Nr. | Carbonsäure-anhydrid (II) | Acylcyanid (I) | Ausbeute (%) | Siedepunkt (Kp.) Schmelzpunkt (Fp.) |
|---|---|---|---|---|
| 4 | 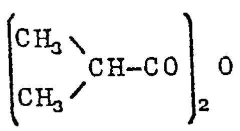 | 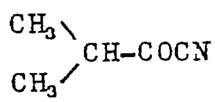 | 89 | Kp. 116-118°C |
| 5 | 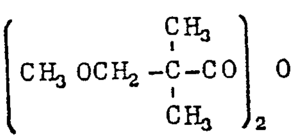 | 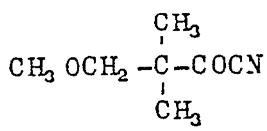 | 91 | Kp. 62-64°C/ 16 mbar |
| 6 | 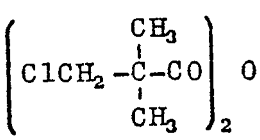 | 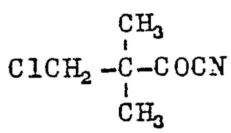 | 88 | Kp. 62-65°C/ 16 mbar |
| 7 | 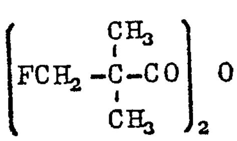 | 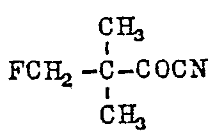 | 94 | Kp. 147-151°C |
| 8 | 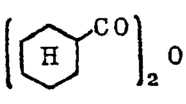 | 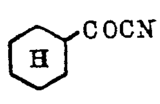 | 82 | Kp. 79-81°C/ 18 mbar |
| 9 | 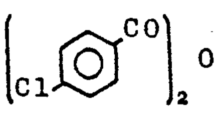 | 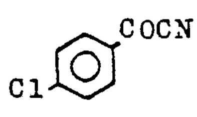 | 94 | Kp. 114-116°C/ 18 mbar Fp. 40°C |

Tabelle 1 (Fortsetzung)

| Bsp. Nr. | Carbonsäure-anhydrid (II) | Acylcyanid (I) | Ausbeute (%) | Siedepunkt (Kp.) Schmelzpunkt (Fp.) |
|---|---|---|---|---|
| 10 | 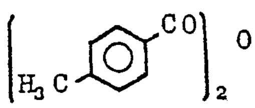 | 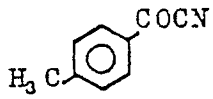 | 92 | Kp. 112-114°C/ 18 mbar<br>Fp. 50-51°C |
| 11 | 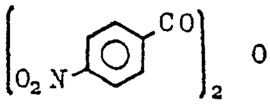 | 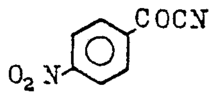 | 84 | Fp.: 116,5°C |
| 12 | 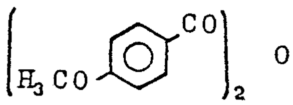 | 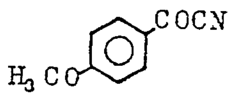 | 99 | Kp. 102-104°C/ 0,1 mbar<br>Fp.: 60-61°C |

Beispiel 12a

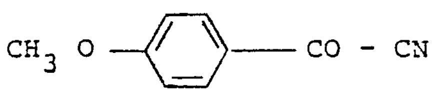

143 g (0,5 Mol) 4-Methoxy-benzoesäureanhydrid, 49,5 g (0,5 Mol) Trimethylsilylcyanid und 1 ml Triethylamin werden gemischt und 1 Stunde bei 40-50 °C gerührt. Die Reaktionsprodukte werden anschließend destillativ aufgearbeitet.

Ausbeute gemäß Gaschromatogramm : 79,3 g 4-Methoxybenzoylcyanid (≙ 98,5 % d. Th.) ; Siedepunkt 102-104 °C/0,1 mbar, Schmelzpunkt 60-61 °C. Außerdem erhält man die entsprechende Menge an 4-Methoxybenzoesäure-trimethylsilylester (Ausbeute : 98 % d. Th.).

Analog zu Beispiel 12a können auch die in der folgenden Tabelle 2 aufgeführten Acylcyanide hergestellt werden :

Tabelle 2

| Beispiel Nr. | Acylcyanid (I) | Ausbeute (%) | Siedepunkt | Schmelzpunkt |
|---|---|---|---|---|
| 1a | 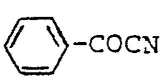 | 97 | 91-92°C/ 14mbar | 31-32°C |
| 9a | 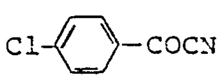 | 93 | 114-116°C/ 18mbar | 40°C |
| 11a | 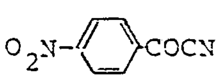 | 81 | - | 116.5°C |

Tabelle 2 (Fortsetzung)

| Beispiel Nr. | Acylcyanid (I) | Ausbeute (%) | Siedepunkt | Schmelzpunkt |
|---|---|---|---|---|
| 10a | 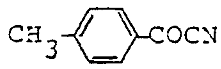 | 91 | 112-114 °C/ 18mbar | 50-51 °C |
| 3a | $(CH_3)_3C-COCN$ | 98 | 121 °C/ 990mbar | - |

Beispiel 3b

$(CH_3)_3C—CO—CN$

93 g (0,5 Mol) Pivalinsäureanhydrid und 1 g Natriumcyanid werden vorgelegt und 49,5 g (0,5 Mol) Trimethylsilylcyanid werden zugetropft. Die Reaktionsmischung wird destillativ aufgearbeitet.

Ausbeute : 56 g Pivaloylcyanid (≙ 98.9 % d. Th.) ;

Siedepunkt : 121-125 °C.

Daneben erhält man Pivalinsäure-trimethylsilylester in entsprechender Menge (99 % d. Th.).

Beispiel 1b

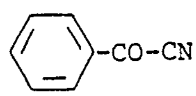

113 g (0.5 Mol) Benzoesäureanhydrid und 1 g 1.4-Diazabicyclo [2.2.2]-octan werden vorgelegt ; bei max. 40 °C werden 49.5 g (0.5 Mol) Trimethylsilylcyanid zugetropft. Die Mischung wird 4 Stunden bei Raumtemperatur gerührt und ergibt nach Gaschromatogramm folgende Ausbeuten : 88.5 % d. Th. Benzoylcyanid und 97.0 % d. Th. Benzoesäuretrimethylsilylester.

Beispiel 12b

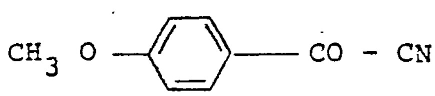

143 g (0,5 Mol) 4-Methoxy-benzoesäureanhydrid, 1 g Natriumcyanid (NaCN) und 49.5 g (0.5 Mol) Trimethylsilylcyanid werden zusammengegeben und 30 Minuten bei Raumtemperatur gerührt. Die Ausbeuten betragen nach Gaschromatogramm : 98.5 % d. Th. 4-Methoxy-benzoylcyanid und 99.0 % d. Th. 4-Methoxy-benzoesäure-trimethylsilylester.

**Patentansprüche**

1. Verfahren zur Herstellung von Acylcyaniden der Formel

$$R-\overset{\overset{O}{\|}}{C}-CN \qquad (I)$$

in welcher R für gegebenenfalls substituiertes Alkyl mit 3 bis 8 Kohlenstoffatomen, gegebenenfalls substituiertes Cycloalkyl mit 3 bis 12 Kohlenstoffatomen, gegebenenfalls substituiertes Aryl oder eine

gegebenenfalls substituierten 5- oder 6-gliedrigen heterocyclischen Rest steht, der zusätzlich noch mit einem Benzolring anelliert sein kann,
dadurch gekennzeichnet, daß man Carbonsäureanhydride der Formel

$$R-\overset{\overset{\displaystyle O}{\|}}{C}-O-\overset{\overset{\displaystyle O}{\|}}{C}-R \qquad (II)$$

in welcher R die oben angegebene Bedeutung hat, mit Trimethylsilylcyanid, $(CH_3)_3Si-CN$ (III), gegebenenfalls in Gegenwart eines Katalysators und gegebenenfalls in Gegenwart eines Verdünnungsmittels, bei Temperaturen zwischen 50 und 250 °C umsetzt.

2. Verfahren nach Anspruch 1, dadurch gekennzeichnet, daß man die Umsetzung bei Temperaturen zwischen 70 und 230 °C, insbesondere zwischen 80 und 200 °C, durchführt.

3. Verfahren nach Anspruch 1, dadurch gekennzeichnet, daß man Carbonsäureanhydrid (II) und Trimethylsilylcyanid (III) in stöchiometrischen Mengen einsetzt.

4. Verfahren nach Anspruch 1, dadurch gekennzeichnet, daß man als Carbonsäureanhydrid (II) Benzoesäureanhydrid oder Pivalinsäureanhydrid einsetzt.

5. Verfahren nach Anspruch 1, dadurch gekennzeichnet, daß die Umsetzung in Gegenwart einer katalytischen Menge einer Lewis-Säure durchgeführt wird.

6. Verfahren nach Anspruch 1, dadurch gekennzeichnet, daß die Umsetzung in Gegenwart einer katalytischen Menge einer Base durchgeführt wird.

**Claims**

1. Process for the preparation of acyl cyanides of the formula

$$R-\overset{\overset{\displaystyle O}{\|}}{C}-CN \qquad (I)$$

in which R represents optionally substituted alkyl with 3 to 8 carbon atoms, optionally substituted cycloalkyl with 3 to 12 carbon atoms, optionally substituted aryl or an optionally substituted 5- or 6-membered heterocyclic radical which additionally can be fused to a benzene ring,
characterised in that carboxylic acid anhydrides of the formula

$$R-\overset{\overset{\displaystyle O}{\|}}{C}-O-\overset{\overset{\displaystyle O}{\|}}{C}-R \qquad (II)$$

in which R has the abovementioned meaning, are reacted with trimethylsilyl cyanide, $(CH_3)_3Si-CN$ (III), if appropriate in the presence of a catalyst and if appropriate in the presence of a diluent, at temperatures between 50 and 250 °C.

2. Process according to Claim 1, characterized in that the reaction is carried out at temperatures between 70 and 230 °C, in particular between 80 and 200 °C.

3. Process according to Claim 1, characterised in that carboxylic acid anhydride (II) and trimethylsilyl cyanide (III) are used in stoichiometric amounts.

4. Process according to Claim 1, characterised in that benzoic acid anhydride or pivalic acid anhydride are used as the carboxylic acid anhydride (II).

5. Process according to Claim 1, characterised in that the reaction is carried out in the presence of a catalytic amount of a Lewis acid.

6. Process according to Claim 1, characterised in that the reaction is carried out in the presence of a catalytic amount of a base.

**Revendications**

1. Procédé de préparation des cyanures d'acyle de formule

$$R-\overset{\overset{\displaystyle O}{\|}}{C}-CN \qquad (I)$$

dans laquelle R représente un groupe alkyle en $C_3$-$C_8$ éventuellement substitué, cycloalkyle en $C_3$-$C_{12}$ éventuellement substitué, aryle éventuellement substitué ou un reste hétérocyclique à 5 ou 6 chaînons

éventuellement substitué et qui peut encore être condensé avec un noyau benzénique, caractérisé en ce que l'on fait réagir des anhydrides d'acides carboxyliques de formule

$$R-\overset{O}{\overset{\|}{C}}-O-\overset{O}{\overset{\|}{C}}-R \qquad (II)$$

dans laquele R a les significations indiquées ci-dessus, avec le cyanure de triméthylsilyle, $(CH_3)_3Si\text{-}CN$ (III), éventuellement en présence d'un catalyseur et éventuellement en présence d'un diluant, à des températures de 50 à 250 °C.

2. Procédé selon la revendication 1, caractérisé en ce que l'on effectue la réaction à des températures de 70 à 230 °C, plus spécialement de 80 à 200 °C.

3. Procédé selon la revendication 1, caractérisé en ce que l'on met en œuvre l'anhydride d'acide carboxylique II et le cyanure de triméthylsilyle III en quantités stoechiométriques.

4. Procédé selon la revendication 1, caractérisé en ce que l'anhydride d'acide carboxylique II mis en œuvre est l'anhydride benzoïque ou l'anhydride pivalique.

5. Procédé selon la revendication 1, caractérisé en ce que l'on effectue la réaction en présence d'une quantité catalytique d'un acide de Lewis.

6. Procédé selon la revendication 1, caractérisé en ce que l'on effectue la réaction en présence d'une quantité catalytique d'une base.